Europäisches Patentamt

European Patent Office

Office européen des brevets

(19)

(11) Publication number: **0 416 670 B1**

(12) **EUROPEAN PATENT SPECIFICATION**

(45) Date of publication of patent specification: **27.04.94**   (51) Int. Cl.5: **C08B 37/00**, A23L 1/308, A23L 1/19

(21) Application number: **90202070.0**

(22) Date of filing: **27.07.90**

(54) **Low-calorie polydextrose derivatives.**

(30) Priority: **07.09.89 NL 8902243**

(43) Date of publication of application:
**13.03.91 Bulletin 91/11**

(45) Publication of the grant of the patent:
**27.04.94 Bulletin 94/17**

(84) Designated Contracting States:
**AT BE CH DE DK ES FR GB GR IT LI LU NL SE**

(56) References cited:
EP-A- 0 271 963
US-A- 2 962 419
US-A- 3 973 049

**FOOD TECHNOLOGY, January 1988, pages 84-90; R.G. LaBARGE: "The search for a low-caloric oil"**

**Encyclopedia of Polymer Science and Engineering, vol. 7, John Wiley & Sons, New York 1985, page 276**

**F H Mattson and R A Volpenhein, Journal of Lipid Research, vol. 13 (1972), pages 325-327**

(73) Proprietor: **COÖPERATIEVE VERENIGING SUIKER UNIE U.A.**
**Zuilenstraat 100**
**NL-4814 ND Breda(NL)**

(72) Inventor: **Vianen, Gerardus Maria**
**Langeveld 25**
**NL-4702 XW Roosendaal(NL)**
Inventor: **Koerts, Kees**
**Buntlaan 43**
**NL-3971 JB Driebergen(NL)**
Inventor: **Kuzee, Hendrika Cornelia**
**Kanaalstraat 57**
**NL-4388 BK Oost-Souburg(NL)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

EP 0 416 670 B1

**Description**

The invention relates to low-calorie polydextrose derivatives.

As is known, there is an ever growing interest in food products having a low calorific value. For example, these products may be used as no-calorie sweeteners instead of carbohydrates and as no-calorie or low-calorie fat substitutes instead of fats.

As regards the fat substitutes, many types of products are known from the prior art. Examples of these are mentioned below.

a) Polydextrose products; these are low-calorie materials (approximately 1 kcal/g) which are capable of replacing the fat constituent in certain preparations. The most known polydextrose product is polydextrose A produced by Pfizer Inc., USA, which is a condensation product of glucose, sorbitol and citric acid; see US patent specifications 3,766,165 and 3,876,794.

b) Sucrose polyesters; for use as a fat substitute, this type of product is usually composed of a mixture of hexa-, hepta-and/or octa-esters of sucrose with fatty acids containing 12-18 carbon atoms. Such esters cannot be broken down in the gastro-intestinal tract and for this reason are designated as no-calorie products. Apart from the fact that said esters lower the cholesterol content of the blood, they do, however, remove fat-soluble vitamins and hormonal substances at the same time. In addition, it is particularly disadvantageous that these polyesters, which pass through the gastro-intestinal tract unaltered, may give rise to "anal leakage".

The sucrose polyesters have the advantage that they are able to replace fats and oils in virtually all applications. The production and use of the sucrose polyesters is discussed in many patent specifications, such as the US patent specifications 3,600,186, 3,963,699, 4,517,360, 4,518,772, 4,005,195, 4,005,196, 4,034,083, 4,368,213, 4,461,786, 4,446,165 and 4,680,184, European patent specifications 233,856, 236,288, 034,858 and 235,836, and also British patent application 2,161,806.

c) Further fat substitutes are known which are based on egg and milk proteins, which proteins acquire the desired "fat" properties by undergoing a physical treatment (micro-particulation). However, such products based on proteins are not heat-resistant and cannot therefore be used for deep frying or baking.

d) In addition, there are fat substitutes based on dextrin and maltodextrin. Many types of commercial products are composed of these, such as

    1. Maltrin MO40® produced by GRAIN PROCESSING CO. USA,
    2. N-oil®; a tapioca dextrin produced by NATIONAL STARCH, USA,
    3. Nutrifat C® produced by REACH ASSOCIATES, USA,
    4. Olestrin® produced by REACH ASSOCIATES, USA, and
    5. Paselli SA2® produced by AVEBE, Holland.

However, these datrins have a calorific value of 4 kcal/g. Since these products are usually used in a 25% aqueous solution, a fat having a calorific value of 9 kcal/g can be replaced by such a product having a calorific value of 1 kcal/g.

e) Finally, fat substitutes based on polyglycerol esters, propoxylated glycerol esters, polysiloxanes, jojoba oil, glycerol ethers and polycarboxylic esters have been proposed.

Further it is pointed at R.G. LaBarge, Food Technology, January 1988, pages 84-90 referring many of the above-mentioned products. It is also mentioned therein that many effects involving the development of fat substitutes relate to carbohydrate fatty acid esters, in particular sucrose polyester (SPE). Other mentioned fat substitutes are polycarboxylic acid esters, sterically hindered esters, medium-chain triglycerides (MTC) and polyglycerins as well as ethers. However, at the end of the article it is clearly pointed out that the combination of desirable fatlike properties with decreased absorbability or digestibility cannot be predicted with any degree of accuracy. Accordingly, selection of non-digestible fats suitable for food compositions is still a "hit or miss" proposition.

It has been found, however, that, owing to their properties, the abovementioned types of fat substitutes cannot function as fat substitutes under all circumstances or have too high calorific value.

The object of the invention is therefore to develop a virtually no-calorie fat substitute means which approaches the natural fats and oils as regards the properties thereof, with the result that the product can be used universally.

Surprisingly, it was found that the abovementioned object can be achieved with polydextrose derivatives as fat substitutes, which polydextrose derivatives are esters, on the one hand, of "polydextrose" products and, on the other hand, of fatty acids advantageously containing 8-22 carbon atoms.

The term "polydextrose" products is understood to mean, inter alia, the products described in the US patent specifications 3,766,165 and 3,876,794. More particularly, the term "polydextrose" product is

2

understood to mean a condensation product which is made up of at least one saccharide, one polyol and a polycarboxylic acid which functions as a catalyst. As saccharide, mention is advantageously made of glucose, maltose and maltotriose, preferably glucose. At the same time, the glucose can be used in the form of the anhydrate or the monohydrate. The expression "polyol" is understood to mean an acceptable product in the food industry such as, for example, glycerol, erythritol, xylitol, mannitol, galactitol and preferably, sorbitol. The polycarboxylic acid used is a polycarboxylic acid which is acceptable in the food industry, such as fumaric acid, tartaric acid, the terephthalic acids, succinic acid, adipic acid, itaconic acid or the anhydrides of the last three acids and, preferably, citric acid.

As stated, "polydextrose" products are condensation products of mixtures of the abovementioned saccharide, polycarboxylic acid and polyol. Examples of mixtures to be reacted which result in "polydextrose" products are:

- 70-95.4% by weight, advantageously 85.5-91.75% by weight of a saccharide, preferably D-glucose;
- 0.1-10% by weight, advantageously 0.25-2.5% by weight, of a polycarboxylic acid, preferably citric acid, and
- 4.5-20% by weight, advantageously 8-12% by weight, of a polyol, preferably sorbitol.

However, the preferably used "polydextrose" product is a condensation product of glucose, sorbitol and citric acid.

The fatty acids which can be used within the scope of the invention include both the saturated fatty acids and the mono-unsaturated or polyunsaturated fatty acids containing 6-24 carbon atoms, preferably 8-22 carbon atoms and more in particular 14-18 carbon atoms. Examples of such fatty acids are palmitic acid, stearic acid, lauric acid, oleic acid, linoleic acid etc. The fatty acids can be used as a mixture of one or more saturated and unsaturated fatty acids containing 8-22 carbon atoms.

The degree of substitution of the polydextrose derivative according to the invention, defined as

$$\frac{\text{mol of fatty acid}}{\text{mol of monosaccharide + mol of polyol,}}$$

may vary within a wide range, for example from 0.1 to 3.5. Advantageously, the degree of substitution has a value of 0.8 to 3.5.

The fatty acid esters of polydextrose according to the invention are novel compounds having unique properties as fat substitutes. More particularly, said compounds according to the invention have the characteristics below:

a) as regards properties, they are similar to those of the natural fats and oils, with the result that the compounds according to the invention can be used universally;

b) the fat properties such as solubility, melting point and the like are achieved even at a relatively low degree of substitution;

c) the starting substances, "polydextrose', and fatty acids, are products which are acceptable in the food industry;

d) owing to the low degree of substitution, the problem of removal of fat-soluble vitamins is less than in the case of the known sucrose polyesters;

e) since the applicant has found that the compounds are not accessible to the enzymes he has investigated because of their complex structure, the compounds according to the invention may be regarded as no-calorie compounds.

In view of the abovementioned favourable characteristics, the fatty acid esters or polydextrose according to the invention can be used in many fields as fat substitutes. Fields of application are, for example, sauces, mayonnaise, ice mixtures and cake mixtures, chocolate, desserts, "toppings", cakes and pastry, margarine, baking and frying oil, deep-frying fat, baked products, and also cosmetics.

The fatty acid esters of polydextrose according to the invention can be prepared by many methods. In addition to solvent-rich methods, solvent-free esterification and transesterification methods can be used. In the working-up, many types of suitable solvents, for example the solvents acceptable in the food industry such as ethanol, isopropanol, hexane, ethyl acetate, methyl ethyl ketone and the like, can be used.

In the embodiment examples below, some possible methods of preparation are mentioned and some fatty acid esters of polydextrose according to the invention are prepared. It is emphasized, however, that the data mentioned in the examples must not be interpreted as restrictive as regards preparation conditions and products prepared.

## Example I

20 g of polydextrose A (Pfizer) were dissolved in 75 ml of pyridine in a 500 ml three-necked round-bottomed flask in an oil bath at 45°C while stirring. Then 148.6 ml of palmitoyl chloride were added dropwise in approximately 20 minutes. The temperature of the oil bath was then raised to 120-125°C, after which the reaction mixture was refluxed for two hours. The reaction mixture was then cooled to 80-90°C and transferred to a conical flask, to which 250 ml of toluene and 350 ml of water were added. The pH of the aqueous solution was approximately 4.5. After the aqueous layer had been separated off, 250 ml of water were again added and the pH was held at a value of 7.5-8.5 for 30 minutes using 5 N NaOH. After the pH had been adjusted to 6.0 using 6 N $H_2SO_4$, the water layer was separated off. The toluene layer was then washed a further three times with 250 ml of water and then evaporated to dryness using a rotary vacuum evaporator. This dried product was dissolved in 100 ml of toluene, after which the solution obtained was added while stirring to 1000 ml methanol at a temperature of 50°C. This solution was slowly cooled to 20°C. The precipitate formed was filtered off and then recrystallized once more from methanol. The solid material obtained was dried in a vacuum oven at 40°C and 10 mbar. The dried material was then dissolved in hexane in a concentration of 10 % and bleached three times with 5% by weight active carbon, type CN1 (Norit). After this bleaching, the hexane solution was evaporated to dryness and the solid material was dried to a constant weight in a vacuum oven (10 mbar, 40°C).

The yield of the final product having a fatty acid content of 0.1% by weight and a methyl palmitate content of 0.3% by weight was 113.6 g. The melting point of the final product was 48-50°C. From the yield obtained a degree of substitution of 3.2-3.4 was calculated.

## Example II

The procedure was as described in Example I, provided that 117 ml of lauroyl chloride was now used instead of 148.6 ml of palmitoyl chloride and that the solution in methanol was cooled to -15°C instead of 20°C. The yield of final product was 90.8 g. The final product was liquid at 20°C and contained 0.1% by weight of fatty acid and 0.2% by weight of methyl laurate. The degree of substitution thereof was 3.4.

## Example III

The procedure was as described in Example I, provided that 157.6 ml of oleyl chloride was now used instead of 148.6 ml of palmitoyl chloride and that the solution in methanol was cooled to -15°C instead of to 20°C. The yield of final product was 126.2 g. The final product was liquid at 20°C and contained 0.3% by weight of fatty acid and 0.7% by weight of methyl oleate. The degree of substitution thereof was 3.4.

## Example IV

The procedure was as described in Example 1, provided that 74.3 ml of palmitoyl chloride were now added to 40 g of polydextrose in 150 ml of pyridine. The fatty acid content of the dried toluene / intermediate product was determined with the aid of HPLC (High Performance Liquid Chromatography). This content proved to be 21.2% by weight of palmitic acid. The product was dissolved in 800 ml of MEK (methyl ethyl ketone), after which $Ca(OH)_2$ was added in a 20% excess (compared with the fatty acid present). The pH was held at a constant value of 7.0 for 2 hours. The calcium cake was then filtered off and 150 ml of water were added to the filtrate. The pH value was then adjusted to 6.0 using 6 N $H_2SO_4$. The MEK layer was then evaporated to dryness in a rotary vacuum evaporator. The solid material was bleached in the manner described in Example I and dried. The yield of final product was 50.1 g and it contained 0.1% by weight fatty acid.

The melting point of the final product was 56-58°C and the degree of substitution thereof was approximately 0.8.

## Example V

The procedure was as described in Example IV, provided that 88 ml of lauroyl chloride was now added to 30 g of polydextrose in 112.5 ml of pyridine. The yield of final product was 80 g. The final product contained 1.1% by weight fatty acid and was semi-liquid and semi-solid at 20°C. The degree of substitution thereof was approximately 1.8.

4

## Claims
## Claims for the following Contracting States : AT, BE, CH, DE, DK, FR, GB, GR, IT, LI, LU, NL, SE

1.  Esters of polydextrose with at least one fatty acid, which polydextrose is obtained by reacting a saccharide, a polyol and a polycarboxylic acid which acid functions as a catalyst.

2.  Esters of polydextrose with at least one fatty acid according to Claim 1, characterized in that the fatty acid section contains one or more saturated fatty acids containing 6-24 carbon atoms.

3.  Esters of polydextrose with at least one fatty acid according to Claim 1, characterized in that the fatty acid section contains one or more unsaturated fatty acids containing 8-22 carbon atoms.

4.  Esters of polydextrose with at least one fatty acid according to Claim 2 or 3, characterized in that the fatty acid section contains a mixture of one or more saturated and unsaturated fatty acids containing 8-22 carbon atoms.

5.  Esters of polydextrose with at least one fatty acid according to one or more of Claims 1-4, characterized in that the fatty acid contains 14-18 carbon atoms in the fatty acid section.

6.  Esters of polydextrose with at least one fatty acid according to one or more of Claims 1-5, characterized in that the polydextrose section is obtainable by reacting glucose, sorbitol and citric acid.

7.  Esters of polydextrose with at least one fatty acid according to one or more of Claims 1-6, characterized in that the degree of substitution of the obtained ester has a value in the range from 0.1 to 3.5.

8.  Esters of polydextrose with at least one fatty acid according to Claim 6, characterized in that the degree of substitution of the obtained ester has a value in the range from 0.8 to 3.5.

9.  Food product, characterized by a content of an ester of polydextrose with at least one fatty acid according to one or more of Claims 1-8.

## Claims for the following Contracting State : ES

1.  A process for the preparation of esters of polydextrose with at least one fatty acid by esterifying polydextrose, which polydextrose has been obtained by reacting a saccharide, a polyol and a polycarboxylic acid which acid functions as a catalyst.

2.  Process according to Claim 1, characterized in that the fatty acid section of the obtained ester contains one or more saturated fatty acids containing 6-24 carbon atoms.

3.  Process according to Claim 1, characterized in that the fatty acid section of the obtained ester contains one or more unsaturated fatty acids containing 8-22 carbon atoms.

4.  Process according to Claim 2 or 3, characterized in that the fatty acid section of the obtained ester contains a mixture of one or more saturated and unsaturated fatty acids containing 8-22 carbon atoms.

5.  Process according to one or more of Claims 1-4, characterized in that the fatty acid section of the obtained ester contains fatty acids containing 14-18 carbon atoms.

6.  Process according to one or more of Claims 1-5, characterized in that the polydextrose section of the obtained ester is obtainable by reacting glucose, sorbitol and citric acid.

7.  Process according to one or more of Claims 1-6, characterized in that the degree of substitution of the obtained ester has a value in the range from 0.1 to 3.5.

8.  Process according to Claim 6, characterized in that the degree of substitution of the obtained ester has a value in the range from 0.8 to approximately 3.5.

9.  A process for the production of food products, characterized by mixing an ester obtained according to one or more of Claims 1-8 with a food product.

**Patentansprüche**

**Patentansprüche für folgende Vertragsstaaten : AT, BE, CH, DE, DK, FR, GB, GR, IT, LI, LU, NL, SE**

1.  Polydextroseester mit wenigstens einer Fettsäure, wobei die Polydextrose durch Reaktion eines Saccharids, eines Polyols und einer als Katalysator fungierenden Polycarbonsäure erhalten wird.

2.  Polydextroseester mit wenigstens einer Fettsäure nach Anspruch 1, dadurch gekennzeichnet, daß der Fettsäureanteil eine oder mehrere gesättigte Fettsäuren mit 6-24 Kohlenstoffatomen enthält.

3.  Polydextroseester mit wenigstens einer Fettsäure nach Anspruch 1, dadurch gekennzeichnet, daß der Fettsäureanteil eine oder mehrere ungesättigte Fettsäuren mit 8-22 Kohlenstoffatomen enthält.

4.  Polydextroseester mit wenigstens einer Fettsäure nach Anspruch 2 oder 3, dadurch gekennzeichnet, daß der Fettsäureanteil eine Mischung aus einer oder mehreren gesättigten und ungesättigten Fettsäuren mit 8-22 Kohlenstoffatomen enthält.

5.  Polydextroseester mit wenigstens einer Fettsäure nach einem oder mehreren der Ansprüche 1-4, dadurch gekennzeichnet, daß die Fettsäure 14-18 Kohlenstoffatome in dem Fettsäureanteil enthält.

6.  Polydextroseester mit wenigstens einer Fettsäure nach einem oder mehreren der Ansprüche 1-5, dadurch gekennzeichnet, daß der Polydextroseanteil durch Reaktion von Glucose, Sorbit und Zitronensäure erhältlich ist.

7.  Polydextroseester mit wenigstens einer Fettsäure nach einem oder mehreren der Ansprüche 1-6, dadurch gekennzeichnet, daß der Substitutionsgrad des erhaltenen Esters einen Wert im Bereich von 0.1 bis 3.5 hat.

8.  Polydextroseester mit wenigstens einer Fettsäure nach Anspruch 6, dadurch gekennzeichnet, daß der Substitutionsgrad des erhaltenen Esters einen Wert im Bereich von 0.8 bis 3.5 hat.

9.  Lebensmittel, gekennzeichnet durch einen Gehalt an Polydextroseester mit wenigstens einer Fettsäure nach einem oder mehreren der Ansprüche 1-8.

**Patentansprüche für folgenden Vertragsstaat : ES**

1.  Verfahren zur Herstellung von Polydextroseestern mit wenigstens einer Fettsäure durch Veresterung von Polydextrose, wobei die Polydextrose durch Reaktion eines Saccharids, eines Polyols und einer als Katalysator fungierenden Polycarbonsäure erhalten wurde.

2.  Verfahren nach Anspruch 1, dadurch gekennzeichnet, daß der Fettsäureanteil des erhaltenen Esters eine oder mehrere gesättigte Fettsäuren mit 6-24 Kohlenstoffatomen enthält.

3.  Verfahren nach Anspruch 1, dadurch gekennzeichnet, daß der Fettsäureanteil des erhaltenen Esters eine oder mehrere ungesättigte Fettsäuren mit 8-22 Kohlenstoffatomen enthält.

4.  Verfahren nach Anspruch 2 oder 3, dadurch gekennzeichnet, daß der Fettsäureanteil des erhaltenen Esters eine Mischung aus einer oder mehreren gesättigten und ungesättigten Fettsäuren mit 8-22 Kohlenstoffatomen enthält.

5.  Verfahren nach einem oder mehreren der Ansprüche 1-4, dadurch gekennzeichnet, daß der Fettsäureanteil des erhaltenen Esters Fettsäuren mit 14-18 Kohlenstoffatomen enthält.

6.  Verfahren nach einem oder mehreren der Ansprüche 1-5, dadurch gekennzeichnet, daß der Polydextroseanteil des erhaltenen Esters durch Reaktion von Glucose, Sorbit und Zitronensäure erhältlich ist.

**7.** Verfahren nach einem oder mehreren der Ansprüche 1-6, dadurch gekennzeichnet, daß der Substitutionsgrad des erhaltenen Esters einen Wert im Bereich von 0.1 bis 3.5 hat.

**8.** Verfahren nach Anspruch 6, dadurch gekennzeichnet, daß der Substitutionsgrad des erhaltenen Esters einen Wert im Bereich von 0.8 bis ungefähr 3.5 hat.

**9.** Verfahren zur Herstellung von Lebensmitteln, gekennzeichnet durch Mischen eines nach einem oder mehreren der Ansprüche 1-8 erhaltenen Esters mit einem Lebensmittel.

**Revendications**
**Revendications pour les Etats contractants suivants : AT, BE, CH, DE, DK, FR, GB, GR, IT, LI, LU, NL, SE**

**1.** Esters de polydextrose avec au moins un acide gras, polydextrose que l'on obtient en faisant réagir un saccharide, un polyol et un acide polycarboxylique, acide qui agit en tant que catalyseur.

**2.** Esters de polydextrose avec au moins un acide gras selon la revendication 1, caractérisés en ce que la section acide gras contient un ou plusieurs acides gras saturés contenant 6 à 24 atomes de carbone.

**3.** Esters de polydextrose avec au moins un acide gras selon la revendication 1, caractérisés en ce que la section acide gras contient un ou plusieurs acides gras insaturés contenant 8 à 22 atomes de carbone.

**4.** Esters de polydextrose avec au moins un acide gras selon la revendication 2 ou 3, caractérisés en ce que la section acide gras contient un mélange d'un ou plusieurs acides gras saturés ou insaturés contenant 8 à 22 atomes de carbone.

**5.** Esters de polydextrose avec au moins un acide gras selon une ou plusieurs des revendications 1 à 4, caractérisés en ce que l'acide gras contient 14 à 18 atomes de carbone dans la section acide gras.

**6.** Esters de polydextrose avec au moins un acide gras selon une ou plusieurs des revendications 1 à 5, caractérisés en ce que l'on peut obtenir la section polydextrose en faisant réagir du glucose, du sorbitol et de l'acide citrique.

**7.** Esters de polydextrose avec au moins un acide gras selon une ou plusieurs des revendications 1 à 6, caractérisés en ce que le degré de substitution de l'ester obtenu a une valeur dans la gamme de 0,1 à 3,5.

**8.** Esters de polydextrose avec au moins un acide gras selon la revendication 6, caractérisés en ce que le degré de substitution de l'ester obtenu a une valeur dans la gamme de 0,8 à 3,5.

**9.** Produit alimentaire, caractérisé par une teneur d'un ester de polydextrose avec au moins un acide gras selon une ou plusieurs des revendications 1 à 8.

**Revendications pour l'Etat contractant suivant : ES**

**1.** Procédé de préparation d'esters de polydextrose avec au moins un acide gras en estérifiant un polydextrose, polydextrose que l'on a obtenu en faisant réagir un saccharide, un polyol et un acide polycarboxylique, acide qui agit en tant que catalyseur.

**2.** Procédé selon la revendication 1, caractérisé en ce que la section acide gras de l'ester obtenu contient un ou plusieurs acides gras saturés contenant 6 à 24 atomes de carbone.

**3.** Procédé selon la revendication 1, caractérisé en ce que la section acide gras de l'ester obtenu contient un ou plusieurs acides gras insaturés contenant 8 à 22 atomes de carbone.

**4.** Procédé selon la revendication 2 ou 3, caractérisé en ce que la section acide gras de l'ester obtenu contient un mélange d'un ou plusieurs acides gras saturés et insaturés contenant 8 à 22 atomes de carbone.

**5.** Procédé selon l'une ou plusieurs des revendications 1 à 4, caractérisé en ce que la section acide gras de l'ester obtenu contient des acides gras contenant 14 à 18 atomes de carbone.

**6.** Procédé selon l'une ou plusieurs des revendications 1 à 5, caractérisé en ce que l'on peut obtenir la section polydextrose de l'ester obtenu en faisant réagir du glucose, du sorbitol et de l'acide citrique.

**7.** Procédé selon une ou plusieurs des revendications 1 à 6, caractérisé en ce que le degré de substitution de l'ester obtenu a une valeur dans la gamme de 0,1 à 3,5.

**8.** Procédé selon la revendication 6, caractérisé en ce que le degré de substitution de l'ester obtenu a une valeur dans la gamme de 0,8 à approximativement 3,5.

**9.** Procédé pour la production de produits alimentaires, caractérisé par le mélange d'un ester obtenu selon l'une ou plusieurs des revendications 1 à 8 avec un produit alimentaire.